# EUROPEAN PATENT APPLICATION

(11) **EP 2 918 963 A1**
(43) Date of publication of application: **16.09.2015**
(21) Application number: 14275061.1
(22) Date of filing: 14.03.2014
(51) Int. Cl.: G01B 5/20, G01B 5/004, A61B 5/107, A61B 5/00, A42C 2/00

(54) **Method of measuring the contours of a person's head used for manufacturing a custom fit liner of an optical display helmet**

(71) Applicant: BAE Systems PLC, London SW1Y 5AD (GB)
(72) Inventor: The designation of the inventor has not yet been filed
(74) Representative: BAE SYSTEMS plc Group IP Department

(57) **Abstract**

A method of generating a topographic map of a region of a body for the manufacture of a body fitting article to be fitted to the region of the body. The method allows for the resultant topographical map of the body feature to be coupled to an anatomical and functional datum on the body. The method involves use of a contact probe which is configured to generate positional data which defines the surface it is drawn across. The method comprises the steps of urging a contact probe towards the outer surface of the body such that the contact probe is touching the outer surface of the body or separated from the outer surface of the body only by a barrier layer which is flattened against the outer surface of the body by the probe. The contact probe is drawn over the region of the body where the body fitting article is to be located such that the contact probe generates 3D positional data of the surface of the outer surface of the body in the region of the body where the body fitting article is to be located.

## Description

The present disclosure relates to method of generating a map of a region of a body.

In particular the present disclosure is concerned with a method of, and apparatus for, generating a map of a region of a body for the manufacture of a body fitting article to be fitted to the region of the body.

Head mounted displays and other body mounted technology is known in the art. For example, it is common for pilots of modern military aircraft to be equipped with helmet mounted displays to help them perform their operational duties. The head mounted displays comprise an optical element, for example a visor, and a means for projecting additional information, in the form of graphics and/or text, onto the visor. Such head mounted apparel requires a custom fit liner to comfortably align the user's eyes to the optics during operation. This is especially important for manoeuvres involving "g" loadings, where the helmet could become detached or move relative to the pilot's head. Such misalignment could upset his/her use of the head mounted display, which must be aligned correctly with the user's eye to provide meaningful and accurate presentation of information, especially where the information being displayed is intended to be overlaid on the user's line of sight through the visor to reference the view through the visor.

It is known to measure head shape using a non-contacting laser scan method. However, this method does not make adequate compensation for hair style, volume and stiffness. Poor compensation of the effects of hair has led to discomfort, fit stability and misalignment issues.

Hence a means of providing a body fitting article which is less sensitive to the effects of body hair and results in a more accurately and comfortably fitting helmet liner is highly desirable.

### Summary

According to the present invention there is provided a method and apparatus as set forth in the appended claims. Other features of the invention will be apparent from the dependent claims, and the description which follows.

Accordingly there may be provided a method of generating a topographic map of a region of a body for the manufacture of a body fitting article to be fitted to the region of the body using a contact probe which is configured to generate positional data which defines the surface it is drawn across; the method comprising the steps of : urging a contact probe towards the outer surface of the body such that the contact probe is touching the outer surface of the body or separated from the outer surface of the body only by a barrier layer which is flattened against the outer surface of the body by the probe; and drawing the contact probe over the region of the body where the body fitting article is to be located such that the contact probe generates 3D positional data of the surface of the outer surface of the body in the region of the body where the body fitting article is to be located.

The barrier layer may comprise hair and/or a flexible close fitting material provided over the top of the body region to press body hair towards the body.

The method may further comprise: selecting a subset of the 3D positional data to define the topography of an interface surface of the body fitting article.

The method may further comprise: using the probe to generate the 3D positional data which defines a datum position on the body spaced apart from the region of the body where the body fitting article is to be located by touching the probe onto a pre-determined datum location.

The method may further comprise the steps of: locating a datum feature of the body with a reference fixture while the contact probe is urged towards the outer skin of the body and drawn over the region of the body where the body fitting article is to be located; the pre-determined datum location being the region of the reference feature which contacts the datum feature of the body.

The body fitting article may be a helmet and the region of a body may be a head.

The reference fixture may be an eye reference fixture configured to stabilise and immobilise the subject's head.

The datum feature of the body may be an eye socket.

The pre-determined datum location may be a sighting means with which the subject aligns their sight.

The 3D positional data may be acquired from the contact probe and processed to define the topography of the body region.

There may also be provided a method of manufacturing a body fitting article to be fitted to a region of a body comprising the steps of: generating a topographic map of a region of the body; and forming a region of a body fitting article in dependence upon the generated 3D positional data such that a region of the article conforms to the shape of the region the body where the body fitting article is to be located.

There may also be provided a method of manufacturing a custom fit liner for use in a helmet in dependence upon the topographical map generated by a method of the present disclosure, the liner being configured to align optics carried by the helmet with the eye line of a wearer of the helmet.

There may also be provided apparatus for performing a method of the present disclosure comprising: a contact probe having a contact surface for contacting the outer surface of the body, the contact surface being provided as sphere or truncated sphere; and a positional data transmitter element provided at the geometric centre of the sphere.

The apparatus may further comprise a reference fixture for stabilising and immobilising the region of a body while the contact probe is drawn over the region of the body where the body fitting article is to be located.

Hence there is provided a contact system for measuring a body part to enable the manufacture of a custom fit liner to fit within a standard helmet and thereby align the optical system carried by the helmet with the eyes of the wearer. The use of a contact method to measure user's head shape is configured to be insensitive to the effects of hair style, volume and stiffness.

### Brief Description of the Drawings

Examples of the present disclosure will now be described with reference to the accompanying drawings, in which:
Figure 1 shows a side view of a subject's head with a cross-sectional view of a helmet;
Figure 2 shows a perspective view of a fixture according to the present disclosure with a subject's head in position;
Figure 3 shows a probe according the present disclosure;
Figure 4 shows a cross-sectional view of the fixture shown in Figure 2;
Figure 5 shows a similar view to that in Figure 4, with a cap according to the present disclosure fitted to the subject's head; and
Figure 6 shows a 2D representation of 3D topographical data representing the subject's scalp acquired using the method and equipment according to the present disclosure.

### Detailed Description

The present disclosure relates to a method of generating a topographic map of a region of a body for the manufacture of a body fitting article to be fitted to the region of the body, including a method of collection of positional data required to generate the topographic map. The present disclosure also relates to a method of designing a body part fitting article to be fitted to a region of a body. In the non limiting examples described, the body part is a human head, and the body part fitting article is a helmet. The method and apparatus herein described provide a means to measure the 3-dimensional shape of a head to allow definition of a custom liner to fit a helmet comfortably and accurately over the subject's head.

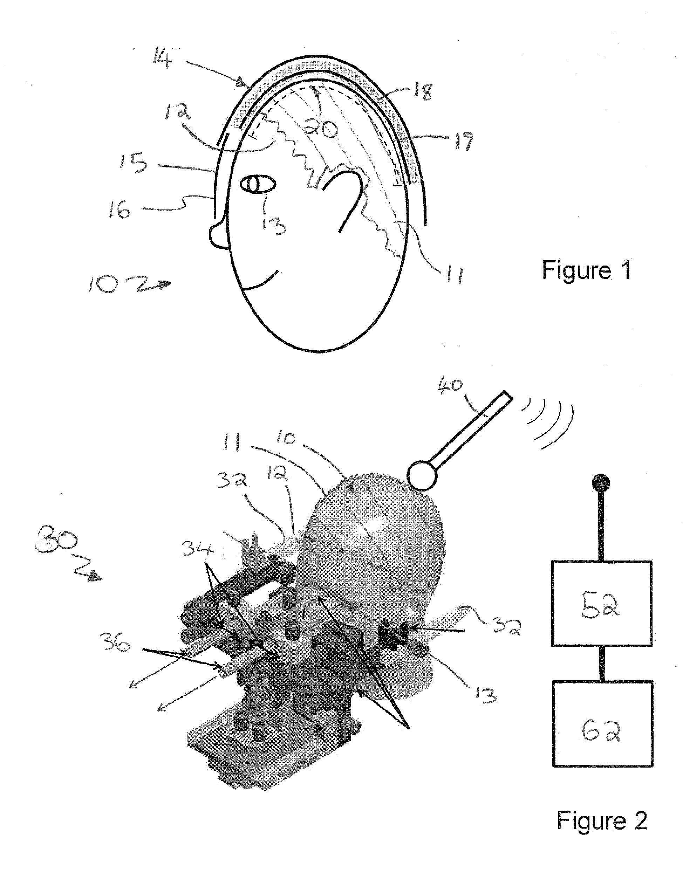

Figure 1 shows a side view of a human subject's head 10, comprising hair 11, skin 12, eyes 13 with a cross-sectional view of a helmet 14 mounted on the head 10. The helmet 14 is fitted with a visor 15 which, as part of its structure, is provided with an optical display 16 to be combined with the subject's view through the visor 15, for example by projection onto a surface of the visor 15 for viewing by the subject. Examples of the visor 15 and optical display 16 and how they may be combined are well known and will not be described in detail in the present disclosure. However, the visor 15 is an integral part of the helmet 14, and the visor 15 must be located correctly relative to the subject's eyes 13 such that the subject can both see through the visor 15 to view his environment, and also have the information from the display 16 aligned correctly with the real world view of his environment through the visor 15. That is to say, the user's eyes and active "exit pupil" of the optical display must be aligned.

According to the method and apparatus of the present disclosure, a lining 18 is provided in the helmet 14 which conforms to the shape of the user's head 10 over a region 20 of the user's head 10 which will thus correctly locate the helmet 14 on the user's head 10. Spaced between the lining 18 and the users head is a "comfort" layer 19, which accommodates minor inconsistencies between the shape of the liner 18 and the subject's head, and may comprise a resilient foam or gel. The region 20, as shown, may span only part of the inner surface of the helmet 14. With such a lining 18 the helmet 14 will be inclined to locate in one position on the subjects head 10, and once fitted in place will be disinclined to move position relative to the head 10.

Figure 2 shows an eye reference fixture 30. The eye reference fixture 30 provides stabilisation features 32 to immobilise the head 10 and eye alignment features 34. The eye alignment features 34 may comprise sighting tubes 36, the axes of which are provided for line of sight and vertical, transverse eye alignment with side sighting blocks for the fore and/or aft position. The eye reference feature 30 may be mounted on any appropriate support structure (for example a table or tripod) that allows the reference fixture 30 to be comfortably located with respect to the subjects head 10 such that it will be possible for the head 10 to be kept static for the duration of the method of the present disclosure.

The sighting tubes 36 fitted to the reference fixture 30 are adjusted to suit the subject's Interpupillary Distance (IPD) prior to alignment. The sighting tubes 36 are configured such that a subject's head 10 may be positioned so the subject can to look through the sighting tubes 36 and the surface of the subject's corneas located at the correct fore and/or aft separation from the tubes 36 back surface. In addition the Line Of Sight (LOS) of the sighting tubes can be adjusted to suit the Frankfurt Plane, chin to brow or any other anatomical reference.

For the avoidance of doubt, and as is well known, the Frankfurt plane (also called the auriculo-orbital plane) defines the normal "comfortable" direction that, under normal circumstances, a human will look/face.

Additional steadies may also be provided as part of the fixture 30 for the positioning of brow, chin and cheek areas for the stabilisation of the subject's head.

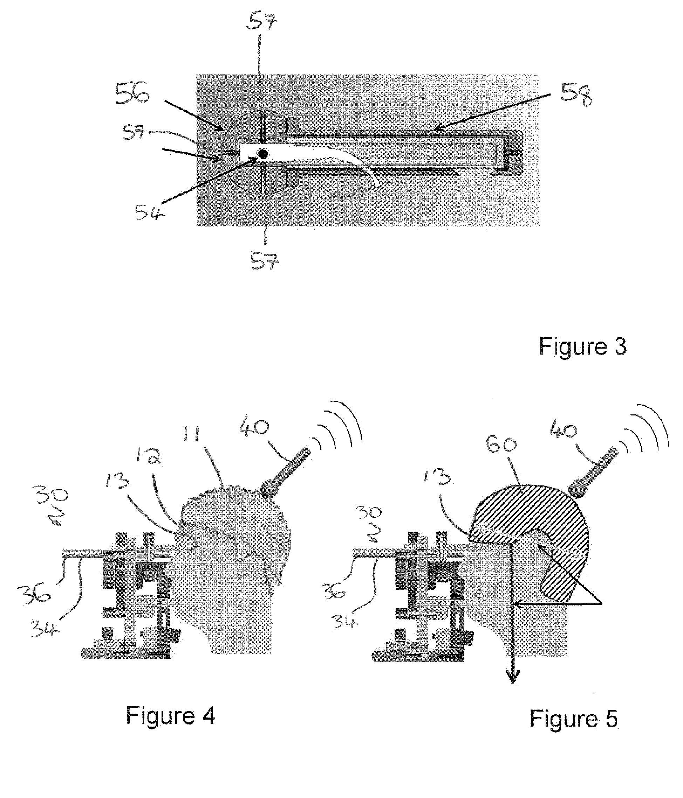

The apparatus of the present disclosure also comprises a probe 40 for collecting data about the shape of the subject's head 10, as shown in Figures 2, 4 and 5 and in more detail in Figure 3. The probe 40 is a contact probe which is configured to generate 3D positional data which defines a surface it is drawn across. The probe 40 may be hand held, and contains an embedded magnetic device 54 that transmits positional data to a receiving unit 52 located at a fixed reference point. Output from the receiver is converted into a stream of 6-Degree Of Freedom (DOF) data providing 3 translations and 3 angular terms.

In the example shown the probe 40 has a spherical measurement end 56, provided as a truncated sphere, and a grip portion 58 which is held by the operator. The positional data transmitter 54 is positioned at the spherical centre of the end 56. In the example shown, grub screws 57 are provided to hold the positional data transmitter 54 in place during assembly. Alternatively, or additionally, the transmitter 54 may also be held in place by an adhesive or cement. The transmitter 54 is positioned at the geometrical centre of the spherical end 56 so that, in use, angular terms defining the orientation of the sphere 56, and hence the position of the transmitter 54, can either be ignored or used for second order corrections during data processing, as required.

The probe end 56 is shaped to minimise contact errors with the underlying outer layer of the body party being probed, for example skin 12, hair 11, and to locally compress and part and the subject's hair 11, where present.

The apparatus and method of the present disclosure may additionally comprise a cap 60 as shown in Figure 5, to assess the contribution of hair 11 for a particular subject. The cap 60 is configured to be fitted over the subject's hair 11. The cap 60 is configured to be tensioned according to the mass supported by the helmet 14 and the reaction of any fitting system such helmet straps. The cap 60 is further configured such that probing the outer surface of the cap 60 will provide an assessment of the offsets required to compensate for the volume, stiffness and style of the subjects hair. The use of the cap is optional.

In operation, a subject places their head 10 in the reference fixture 30 as shown in Figures 2, 4, 5. The sighting tubes 36 fitted to the reference fixture 30 are adjusted to suit the subject's Interpupillary Distance (IPD) prior to alignment. The subject's head 10 is then positioned to look through the sighting tubes and the surface of the corneas located at the correct fore/aft separation from the tubes 36 back surface. In addition the Line Of Sight (LOS) of the sighting tubes 34 can be adjusted to suit the Frankfurt Plane, chin to brow or any other anatomical reference of the subject. Steadies may then be positioned to the brow, chin and cheek areas to stabilise and immobilise the subject's head in the reference fixture 30.

Datum features of the body (in this example, eye socket 13) are located with the reference fixture 30 via the sighting tubes 34 while the contact probe 40 is urged towards (that is to say, placed into contact with) the outer surface 30 of the body 10 and drawn over the region 20 of the body 10 where, in use, the helmet 14 is to be located. The phrase *"outer surface of a body"* is intended to refer to the outer skin, hair etc. of the body being mapped, whatever the outer surface layer may be. In examples where a body part other than a head is being probed for the design, manufacture and fitting of a body fitting article, the *"outer surface of the body"* can equally apply to nails or other features of body, or articles worn on the body.

The probe 40 is urged towards the outer skin 12 of the head (i.e. body part) 10 such that the contact probe is touching the outer skin 12 or separated from the outer skin 12 only by a barrier layer which is flattened against the outer skin 12 by the probe 40. The barrier layer may comprise hair 11 and/or a flexible close fitting material (for example the cap 60) provided over the top of the scanned body region to press body hair towards the body. With the subject suitably immobilised and aligned, the contact probe 40 is drawn over the region of the body 10 where the body fitting article is to be located such that the contact probe 40 generates 3D positional data of the surface of the outer surface of the skin 12 in the region 20 of the body 10 where the body fitting article 12 is to be located. The probe 40 should remain in contact with the subject, and urged towards the subject's outer layer (e.g. skin 12) throughout the process ensuring sufficient data is collected to define a complete surface. That is to say, the probe 40 is passed across the subject's head 10 making sure the spherical surface 56 is in constant contact at all times. The pressure applied to the probe 40 to head 10 interface during the head measurement process is either controlled by operator dexterity or a load cell in the grip 58 of the probe 40 to provide operator feedback. A combination of spherical form and pressure regulation provides the required accurate 3-dimensional map of the subject's scalp without excessive form errors from the presence of hair 11.

In one example, before, or after, probing the head 10, parts of the reference fixture 30 are probed to provide three orthogonal datum's relative to the subjects eyes. That is to say, the probe 40 is used to generate 3D positional data which defines a datum position on the body part 10, where the datum position is spaced apart from the region of the body 10 where the body fitting article (helmet 14) is to be located when worn by the subject. This is achieved by touching the probe 40 onto a pre-determined datum location. The pre-determined datum location is the region of the reference feature which contacts datum feature(s) of the body, which, for example could be either end of the sighting tubes 36.

In another example the receiver 52 may be provided in a fixed (or "known") position relative to the probe 40 and hence acts as a datum position to allow for determining the subject's eye position relative to probed region of the body, thereby obviating the need for probing the reference fixture 30 as defined above. However, the reference fixture 30 may still be probed to provide a confirmatory check in a method employing the receiver 52 as a datum.

In an alternative example, a receiver may be located at the centre of the end 56 of the probe 40, with a transmitter being provided as a separate unit to the probe 40, for example at a fixed (and optionally a "known") position relative to the probe 40.

The contact probing of the head 12, as described above, may return information on body shape which is in part contaminated by the presence of hair 13. If required, the method may also include the step of assessing the contribution of hair for a particular subject by fitting the cap 60 over the hair 13, and tensioning the cap 60 according to the mass supported by the helmet and the reaction of any fitting system such as the nape. Probing the outer surface of the cap 60 in the same way as previously described then provides an assessment of the offsets required to compensate for the volume, stiffness and style of the subjects hair.

During the probing cycle 6-DOF (degrees of freedom) data relating to the location of the probe's spherical centre is continually recorded and stored for post processing. On completion of the head and optional reference fixture probing process, the acquired positional data set contains a cloud of data points which define the relative position of the scanned region of the head to the subject's eye position and/or reference fixture.

The probe motion data, continually recorded during the probing cycle, and datum information is transferred to the receiving unit 52, and then from the receiving unit 52 is communicated to a means for processing the data into a surface map, for example a computer. It may be transferred to Computer Aided Design (CAD) tool 62 for processing into a surface map of the head 10 with respect to the eye datums, as appropriate.

Alternatively, the data transferred to the receiving unit 52 could be transferred directly to a manufacturing tool for production of the body fitting apparel.

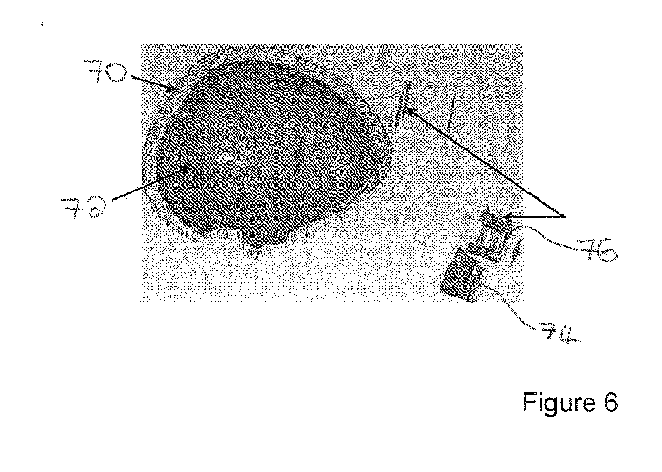

In the construction of a surface map, the positional data must be offset by the radius of the spherical probe to determine the actual head surface topography. Features of the body fitting article must also be allowed for. In the example of a helmet, the thickness of comfort layer 19, lining material and hair thickness must be provided for in the offset in order to produce a correctly fitting helmet. The offset is indicated in Figure 6, where the outer grid 70 indicates the coordinate data collected by the probe, and the lower/solid surface 72 indicates the surface 20 of the head 10. A subset of the 3D positional data obtained may be used to define the topography of the interface surface 20 of the body fitting article 14. The features 74, 76 in Figure 6 represent the pre-determined datum location which, for example, is the end of the sighting tubes 36 distal to the subject's eyes 13.

The data is then used to form a region of a body fitting article (e.g. liner 18 of a helmet 14) in dependence upon the generated 3D positional data, such that the region 20 of the article (helmet) 14 conforms to the shape of the region 20 the body (head 10) where the body fitting article 14 is to be located.

Hence the invention uses a fixture 30 to locate features of the subject (e.g. the subject's eyes) and a roaming contact probe to measure the head shape. The probe 40 is linked to 3-dimensional metrology that records its motion relative to the eye location fixture. The probe locally parts and compresses the hair to minimise contact errors with the underlying skin. The probe can also be used to measure the surface of a head cap placed over the hair.

The above described apparatus may be employed as described in accordance with the present disclosure to measure the 3-dimensional shape of a head 10 (that is to say, a representation of the bald head of a subject) to allow definition of a custom liner 18 to fit a helmet 14 comfortably and accurately over the subject's head 10. That is to say, the method and apparatus of the present disclosure form part of a method of design and manufacture of a bespoke helmet tailored to the needs of an individual user.

The method and apparatus has been developed specifically for use with helmets containing optics (e.g. a helmet with an optical display system that requires alignment to the subject's eyes 13. However, the system (i.e. method an apparatus) could be adapted for alignment with any other anatomical feature, including features on the subject's head and other body parts.

The method and apparatus may be employed in the manufacture of head mounted displays and other body mounted technology.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A method of generating a topographical map of a region of a body for the manufacture of a body fitting article to be fitted to the region of the body
using a contact probe which is configured to generate positional data which defines the surface it is drawn across;
the method comprising the steps of :
urging a contact probe towards the outer surface of the body
such that the contact probe is touching the outer surface of the body or separated from the outer surface of the body only by a barrier layer which is flattened against the outer surface of the body by the probe;
and drawing the contact probe over the region of the body where the body fitting article is to be located
such that the contact probe generates 3D positional data of the surface of the outer surface of the body in the region of the body where the body fitting article is to be located.

2. A method as claimed in claim 1 wherein the barrier layer may comprise hair and/or a flexible close fitting material provided over the top of the body region to press body hair towards the body.

3. A method as claimed in claim 1 wherein the method further comprises: selecting a subset of the 3D positional data to define the topography of a interface surface of the body fitting article.

4. A method as claimed in claim 1, claim 2 or claim 3 wherein the method further comprises: using the probe to generate the 3D positional data which defines a datum position on the body spaced apart from the region of the body where the body fitting article is to be located by touching the probe onto a pre-determined datum location.

5. A method as claimed in claim 4 wherein the method further comprises the steps of: locating a datum feature of the body with a reference fixture while the contact probe is urged towards the outer skin of the body and drawn over the region of the body where the body fitting article is to be located; the pre-determined datum location being the region of the reference feature which contacts the datum feature of the body.

6. A method as claimed in any one of the preceding claims wherein the body fitting article is a helmet; and the region of the body is a head.

7. A method as claimed in claim 6 wherein: the reference fixture is an eye reference fixture configured to stabilise and immobilise the subject's head.

8. A method as claimed in claim 6 or claim 7 wherein: the datum feature of the body is an eye socket.

9. A method as claimed in any one of claims 6 to 8 wherein: the pre-determined datum location is a sighting means with which the subject aligns their sight.

10. A method as claimed in any one of the preceding claims wherein the 3D positional data is acquired from the contact probe and processed to define the topography of the body region.

11. A method of manufacturing a body fitting article to be fitted to a region of a body comprising the steps of: performing the method of any one of claims 1 to 10; and forming a region of a body fitting article in dependence upon the generated 3D positional data such that a region of the article conforms to the shape of the region the body where the body fitting article is to be located.

12. A method of manufacturing a custom fit liner for use in a helmet in dependence upon the topographical map generated by the method of claims 1 to 10, the liner being configured to align optics carried by the helmet with the eye line of a wearer of the helmet.

13. Apparatus for performing a method of any one of claims 1 to 10 comprising: a contact probe having a contact surface for contacting the outer surface of the body, the contact surface being provided as sphere or truncated sphere; and a positional data transmitter element provided at the geometric centre of the sphere.

14. Apparatus as claimed in claim 13 further comprising a reference fixture for stabilising and immobilising the region of a body while the contact probe is drawn over the region of the body where the body fitting article is to be located.
